# EUROPEAN PATENT SPECIFICATION

(11) **EP 4 082 024 B1**
(45) Date of publication and mention of the grant of the patent: **28.01.2026**
(21) Application number: 20838708.4
(22) Date of filing: 09.12.2020
(51) Int. Cl.: G16H 40/63, G16H 20/40, A61B 18/00

(54) **ENERGY-BASED SURGICAL SYSTEMS AND METHODS BASED ON AN ARTIFICIAL-INTELLIGENCE LEARNING SYSTEM**
ENERGIEBASIERTE CHIRURGISCHE SYSTEME UND VERFAHREN, BASIEREND AUF EINEM LERNSYSTEM MIT KÜNSTLICHER INTELLIGENZ
SYSTÈMES ET PROCÉDÉS CHIRURGICAUX À BASE D'ÉNERGIE BASÉS SUR UN SYSTÈME D'APPRENTISSAGE À INTELLIGENCE ARTIFICIELLE

(30) Priority: 23.12.2019 US 201962952803 P
(43) Date of publication of application: 02.11.2022
(73) Proprietor: Covidien LP, Mansfield, MA 02048 (US)
(72) Inventor: JOSEPH, Daniel A., Golden, Colorado 80401 (US); KINGSLEY, Dylan, Broomfield, Colorado 80020 (US)
(74) Representative: Gray, James
(86) International application number: PCT/US2020/063897
(87) International publication number: WO 2021/133549

(56) References cited:
- WO-A2-2019/089919
- CN-A- 109 893 241
- US-A1- 2019 269 384

## Description

### FIELD

The present disclosure relates to energy-based surgical procedures and, more particularly, to energy-based surgical systems and methods based on an artificial-intelligence learning system.

### BACKGROUND

Surgical instruments are utilized to perform various treatments on tissue structures. A surgical forceps, for example, is a plier-like device which relies on mechanical action between its jaws to grasp, clamp, and constrict tissue. Energy-based surgical forceps utilize both mechanical clamping action and energy to treat tissue, such as coagulate, cauterize, and/or seal tissue.

CN 109 893 241 A (UNIV WUHAN, 2019-06-18) discloses an early warning system for bipolar electrocautery for prostate hyperplasia based on FPGA technology. The system is aimed at assisting the doctor in operation and giving early warning information in time to avoid excessive resection and reduce the rate of surgical failure.

While surgical instruments such as energy-based surgical forceps are effective at treating tissue, outcomes of using such instruments may depend on the experience of the clinician using the instruments. For example, vessel sealing is accomplished by subjecting a vessel to a specific energy profile under a specific pressure, and experience of a clinician may affect the outcome. Accordingly, there is continuing interest in improving energy-based surgical systems and methods.

### SUMMARY OF THE INVENTION

The present invention is defined by the appended claims.

### SUMMARY OF RELATED DISCLOSURE

The present disclosure relates to energy-based surgical systems and methods that use an artificial-intelligence learning system. Although portions of the present disclosure discuss particular types of energy-based surgical systems, aspects of the present disclosure are applicable to other types of energy-based surgical systems not expressly described herein. As used herein, the term "distal" refers to the portion that is being described which is further from a user, while the term "proximal" refers to the portion that is being described which is closer to a user. Further, to the extent consistent, any of the aspects or embodiments described herein may be used in conjunction with any or all of the other aspects or embodiments described herein.

In accordance with aspects of the disclosure, a computer implemented method for an energy-based surgical procedure includes accessing an image of tissue of a patient, accessing control parameter values of a generator configured to provide energy based on control parameters, processing the image of the tissue and the control parameter values by an artificial-intelligence learning system to provide an output relating to configuration of the control parameters, providing an indication to a clinician based on the output where the indication indicates whether to maintain the control parameter values, and providing adjusted control parameter values for the generator based on the output of the artificial-intelligence learning system, if the indication indicates not to maintain the control parameter values.

In various embodiments of the method, the output of the artificial-intelligence learning system relates to a predicted outcome of applying the energy based on the control parameter values to the tissue.

In various embodiments of the method, the computer implemented method includes providing the energy for the clinician to apply to the tissue, applying the energy to the tissue using an energy-based surgical instrument, and receiving information from the clinician on an outcome of applying the energy to the tissue.

In various embodiments of the method, the information from the clinician indicates one of: a successful outcome of applying the energy to the tissue or an unsuccessful outcome of applying the energy to the tissue. In various embodiments of the method, the information from the clinician includes at least one of an audio recording or a user-interface selection.

In various embodiments of the method, the image is an image of the tissue captured before the clinician applied the energy to the tissue, and the computer implemented method includes processing the information from the clinician to provide a tag for training the artificial-intelligence learning system, storing training data including the control parameter values and the image of the tissue captured before the clinician applied the energy to the tissue, and training the artificial-intelligence learning system based on the training data and the tag.

In various embodiments of the method, the computer implemented method includes accessing patient information, wherein the artificial-intelligence learning system is configured to provide the output relating to configuration of the control parameters based further on the patient information. In various embodiments of the method, the patient information includes at least one of patient age, moisture of the tissue, hydration of the tissue, or location of the tissue.

In various embodiments of the method, the artificial-intelligence learning system includes a convolutional neural network that processes the image of the tissue.

In various embodiments of the method, providing the adjusted control parameter values for the generator includes automatically adjusting the control parameters, and providing an indication to the clinician that the control parameters have been automatically adjusted.

In accordance with aspects of the present disclosure, an energy-based surgical system includes an image capturing device configured to capture an image of tissue of a patient, and a generator configured to provide energy based on control parameters. The generator is configured to execute instructions to perform a method including accessing the image of the tissue of the patient, accessing control parameter values of the control parameters, processing the image of the tissue and the control parameter values by an artificial-intelligence learning system to provide an output relating to configuration of the control parameters, providing an indication to a clinician based on the output where the indication indicates whether to maintain the control parameter values, and providing adjusted control parameter values for the generator based on the output of the artificial-intelligence learning system, if the indication indicates not to maintain the control parameter values.

In various embodiments of the system, the output of the artificial-intelligence learning system relates to a predicted outcome of applying the energy based on the control parameter values to the tissue.

In various embodiments of the system, the energy-based surgical system includes at least one of a user-interface or a voice recorder, and an energy-based surgical instrument. The generator provides the energy to the energy-based surgical instrument for the clinician to apply to the tissue, and the user-interface and/or the voice recorder receives information from the clinician on an outcome of applying the energy to the tissue.

In various embodiments of the system, the information from the clinician indicates one of: a successful outcome of applying the energy to the tissue or an unsuccessful outcome of applying the energy to the tissue. In various embodiments of the system, the information from the clinician includes at least one of audio recorded by the voice recorder or a selection using the user-interface.

In various embodiments of the system, the image is an image of the tissue captured before the clinician applied the energy to the tissue, and the method performed by executing the instructions in the generator includes processing the information from the clinician to provide a tag for training the artificial-intelligence learning system, storing training data including the control parameter values and the image of the tissue captured before the clinician applied the energy to the tissue, and associating and storing the training data with the tag.

In various embodiments of the system, the energy-based surgical system includes a storage the includes patient information, where the artificial-intelligence learning system is configured to provide the output relating to configuration of the control parameters based further on the patient information. In various embodiments of the system, the patient information includes at least one of patient age, moisture of the tissue, hydration of the tissue, or location of the tissue.

In various embodiments of the system, the artificial-intelligence learning system includes a convolutional neural network that processes the image of the tissue.

In various embodiments of the system, the generator, in providing the adjusted control parameter values, executes the instructions to automatically adjust the control parameters, and provide an indication to the clinician that the control parameters have been automatically adjusted.

### BRIEF DESCRIPTION OF THE DRAWINGS

Various aspects and features of the disclosure are described herein with reference to the drawings wherein:
FIG. 1 is a diagram of an exemplary surgical system including an energy-based surgical instrument and an endoscope, in accordance with aspects of the disclosure;
FIG. 2 is a block diagram of an exemplary artificial intelligence learning system in accordance with aspects of the disclosure;
FIG. 3 is a block diagram of another exemplary artificial intelligence learning system in accordance with aspects of the disclosure;
FIG. 4 is a block diagram of an exemplary data record in accordance with aspects of the disclosure;
FIG. 5 is a perspective view of an exemplary surgical system including an energy-based surgical instrument and a generator, in accordance with aspects of the disclosure;
FIG. 6 is a block diagram of an exemplary generator, in accordance with aspects of the disclosure;
FIG. 7 is a block diagram of an exemplary controller, in accordance with aspects of the disclosure;
FIG. 8 is a flowchart of an exemplary operation for adjusting generator parameters, in accordance with aspects of the disclosure;
FIG. 9 is a perspective view of another exemplary surgical system including another energy-based surgical instrument and generator, in accordance with aspects of the present disclosure; and
FIG. 10 is a perspective view of an exemplary microwave ablation system that includes a generator having a user interface for displaying and controlling ablation patterns, in accordance with aspects of the disclosure.

### DETAILED DESCRIPTION

The present disclosure relates to energy-based surgical systems and methods that use an artificial-intelligence learning system. Although portions of the present disclosure discuss particular types of energy-based surgical systems, aspects of the present disclosure are applicable to other types of energy-based surgical systems not expressly described herein.

As one example of an energy-based surgical procedure, tissue sealing involves heating tissue to liquefy the collagen and elastin in the tissue so that it reforms into a fused mass with significantly-reduced demarcation between the opposing tissue structures. To achieve a tissue seal without causing unwanted damage to tissue at the surgical site or collateral damage to adjacent tissue, the application of energy to tissue is controlled to control the temperature of tissue during the sealing process. To properly seal tissue, a balance should be sustained during the sealing process between sufficient heating to denature proteins and vaporize fluids and poor seal performance. In various situations, implementing such a balance to achieve a proper tissue seal may be based on clinician experience with the energy-based equipment and/or with different conditions of the patient tissue. In accordance with aspects of the present disclosure, and as described below, learning the parameters and outcomes of such clinician experience may be helpful in implementing the proper balance for achieving a successful outcome.

As detailed below, and in accordance with aspects of the present disclosure, the present disclosure involves processing data on configuration of an energy-based surgical system and processing tissue condition information, using an artificial intelligence learning system. Aspects of the present disclosure may also use clinician feedback on the application of a particular energy-based surgical system configuration to a particular tissue to train an artificial intelligence learning system.

The terms "artificial intelligence," "data models," or "system learning" may include, but are not limited to, neural networks, recurrent neural networks (RNN), generative adversarial networks (GAN), Bayesian Regression, Naive Bayes, nearest neighbors, least squares, means, and support vector regression, among other data science and artificial science techniques.

The term "application" may include a computer program designed to perform particular functions, tasks, or activities for the benefit of a user. Application may refer to, for example, software running locally or remotely, as a standalone program or in a web browser, or other software which would be understood by one skilled in the art to be an application. An application may run on the controller 500 or on a user device, including for example, on a mobile device, an IoT device, or a server system.

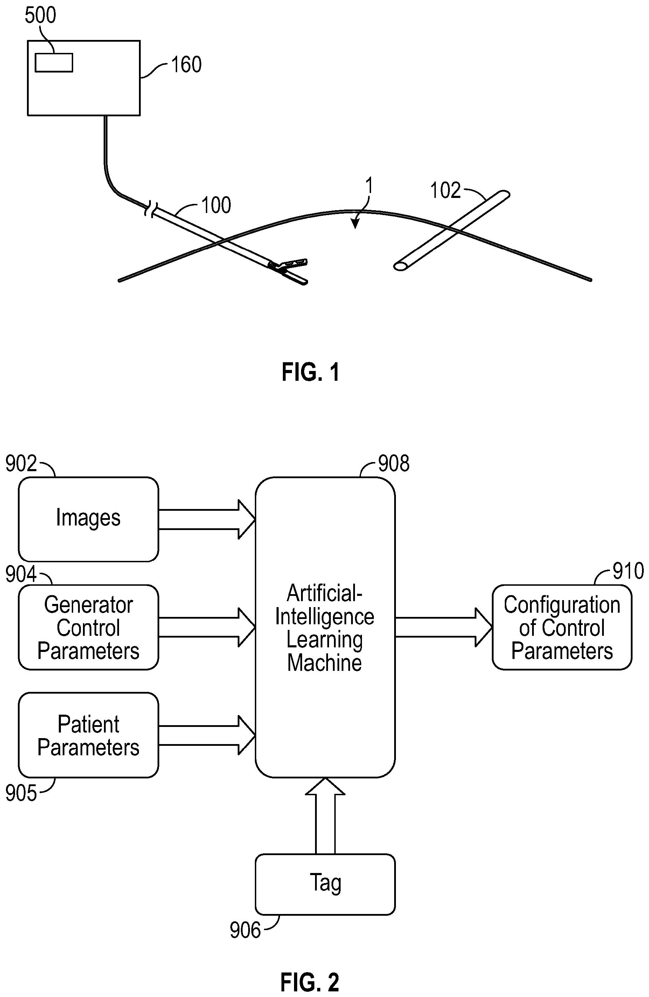

Referring now to FIG. 1, there is shown an embodiment of an energy-based surgical system including a generator 160, an endoscopic surgical forceps 100 for use in connection with endoscopic surgical procedures, and an endoscope 102 for use therewith. The endoscope 102 is used to capture images of the surgical site 1. The present disclosure is applicable where images of a surgical site 1 are captured. Endoscope systems are provided as an example, but it will be understood that such description is exemplary and does not limit the scope and applicability of the present disclosure to other systems and procedures. For example, the image capturing device may be located on the handset of the energy-based surgical instrument. Aspects of the generator 160 will be described in more detail later herein.

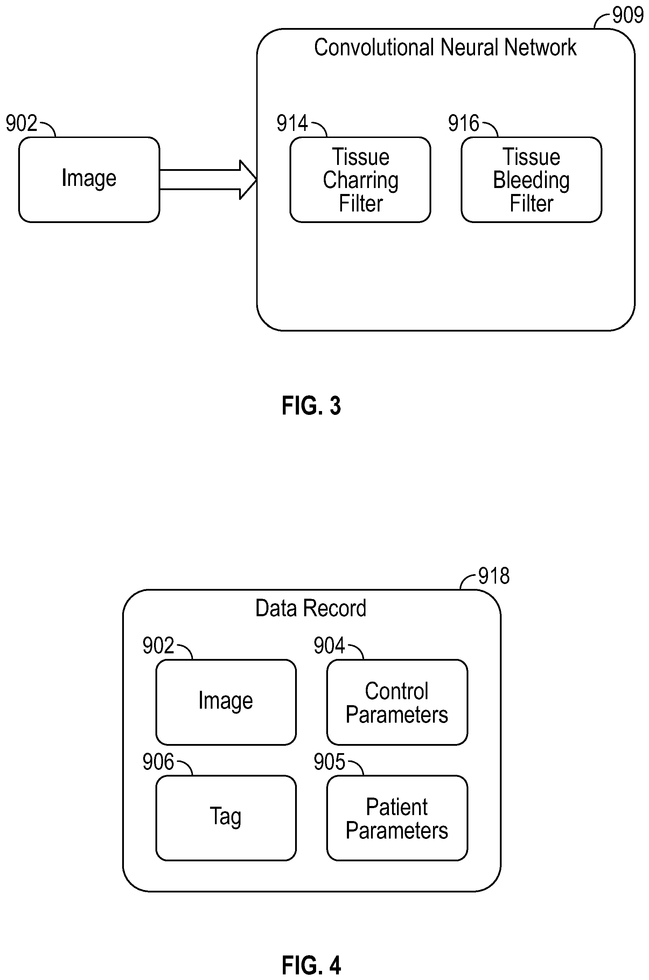

With reference to FIG. 2, a block diagram of an exemplary artificial intelligence learning system 908 is shown in accordance with aspects of the disclosure. As explained in more detail below, the artificial intelligence learning system seeks to utilize clinician experience to determine whether or not a current generator configuration is suitable for a particular patient scenario for achieving a successful outcome. Training of the artificial intelligence learning system 908 may use training data that includes images 902, and current generator control parameter values 904 as inputs to the artificial intelligence learning system 908. In various embodiments, the training data can include patient parameters, which will be discussed in more detail in connection with FIG. 4. The artificial intelligence learning system 908 outputs a configuration of generator control parameters 910 based on the input data and/or outputs an indication regarding whether or not to maintain the current configuration of generator control parameters 910 based on the input data. In various embodiments, the training data may be tagged with a tag 906, which persons skilled in the art will understand as a label that reflects some ground truth about the training data.

The images 902 that are input to the artificial intelligence learning system 908 may show, for example, tissue bleeding and/or tissue charring, among other things. The artificial intelligence learning system 908 may be configured to identify such characteristics in the image 902. With reference to FIG. 3, the artificial intelligence learning system may include a convolutional neural network 909 that has a filter configured to identify tissue charring 914 and/or a filter configured to identify tissue bleeding 916. Persons skilled in the art will understand how to implement and operate such convolutional neural networks 909 and filters. Such characteristics can be identified by the convolutional neural network 909 and can augment/supplement the input data to the artificial intelligence learning system 908.

Referring to FIG. 4, a training data record 918 (FIG. 4) may include an image 902, associated generator control parameters 904, and an associated tag 906, which are used to train the artificial intelligence learning system 908. In various embodiments, the generator control parameters 904 may include, but are not limited to, for example, duration, power, ramp rate, frequency, or other generator parameters. In various embodiments, the training data record 918 may include data relating to patient parameters 905, such as a patient's age, tissue moisture, hydration, and/or tissue location within the patient's body, among other patient characteristics. A person skilled in the art would understand how to determine tissue moisture, such as, for example, as described in U.S. Patent No. 8,961,504. In various embodiments, the data relating to patient parameters 905 may be entered into the system manually, or electronically from the patient's medical records.

In various embodiments, as mentioned above, the image 902, the generator control parameter values 904, and the patient parameters 905, form training data. In various embodiments, the tag 906 relates to the training data 902-905 in a specific way. In particular, with respect to a particular application of energy to a patient based on generator control parameter values, the training data 902-905 is captured before such energy is applied to the patient, and the tag 906 is captured after such energy is applied to the patient. Thus, the training data 902-905 reflects a scenario that a clinician may face before energy is applied to a patient, and the tag 906 reflects whether the outcome of such energy application to the patient was a successful outcome or an unsuccessful outcome. In various embodiments, the clinician may "tag" whether the energy application was successful by using, for example, buttons on the instrument or on the generator or by speech. As another example, the training data may include a tag indicating seal failure weeks later in the patient after they leave hospital. Training the artificial-intelligence learning system in the manner described above results in identifying correlations between the training data 902-905 and the successful and unsuccessful outcomes of the energy-based surgical procedure indicated by the tag 906, and thereby allows the artificial intelligence learning system to learn from clinician experience.

In various embodiments, the "tagging" may be accomplished through audio capture and voice recognition. For example, the instrument or the generator may include a microphone to capture the clinician's speech indicating the outcome of the energy application. Examples of such tagging is shown in Table 1 provided below. In various embodiments, the system can display in text on the generator the words that were recorded from the clinician and that were recognized by speech recognition. In various embodiments, the generator may include indicator lights, which reflect the outcome of the energy application, as indicated by the clinician speech. In various embodiments, the tag 906 that is derived based on the voice recognition may be used as training data for supervised learning of the artificial intelligence learning system 908. It is contemplated that the training may be performed on a separate system, for example, GPU servers, simulation, *etc.,* and the trained network would then be deployed in the energy-based surgical system. In various embodiments, the instrument or generator may include a button to cancel the previous input to correct an incorrect "tag."

**Table 1.**

| **Words spoken by clinician** | **Text recognized** | **Tissue type** | **Size mm** | **Comments** | **Results** | **Illumination color** |
|---|---|---|---|---|---|---|
| 5 mm vessel sticking good seal | 5 mm vessel sticking good seal | Vessel | 5 | Sticking | Successful | Green |
| I hate this machine | I hate this machine | Not specified | Not specified | Dissatisfaction | Unsuccessful | Red |
| I love this machine | I love this machine | Not specified | Not specified | Positive action | Not specified | Green |
| Lung good | Lung good | Lung | Not specified | Not specified | Successful | Green |
| Bowel bad no sticking | Bowel bad no sticking | Bowel | Not specified | No sticking | Successful | Green |

Thus, as mentioned above, the artificial-intelligence learning machine 908 determines correlations between the input data 902-905 and the successful and unsuccessful outcomes of energy-based surgical applications, and thereby learns from clinician experience through the training process. In applying a trained artificial intelligence learning system 908, the controller 500 outputs, from the artificial-intelligence learning system, an indication of whether to maintain a generator's current control parameter values based on tissue image 902, the current generator control values 904, and/or patient parameters 905. In various embodiments, the indicator may be implemented as a visual indication, such as an LED light or a display screen. In various embodiments, the generator 160 provides the visual indication. In various embodiments, the energy-based surgical instrument provides the visual indication. In various embodiments, the image data may be captured before, during, and/or after energy delivery.

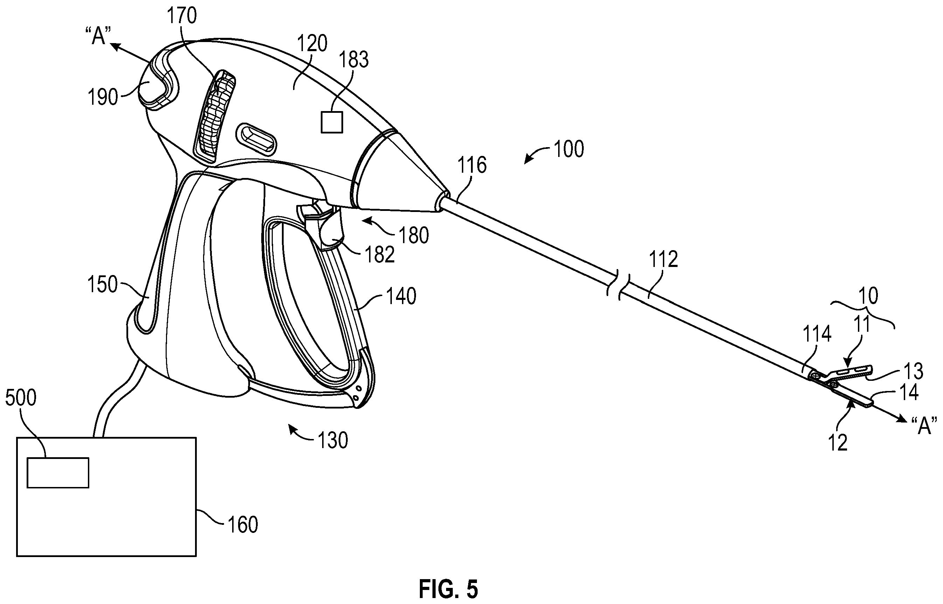

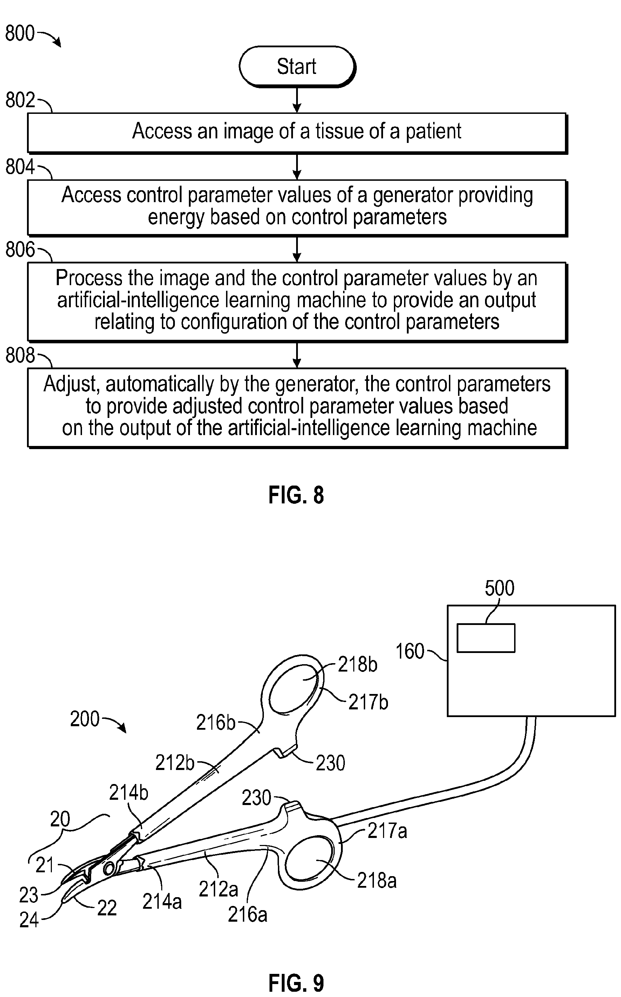

Referring now to FIG. 5, there is shown an exemplary energy-based surgical system, including an endoscopic surgical forceps 100 for use in connection with endoscopic surgical procedures and an generator 160 for use therewith. For the purposes herein, either endoscopic forceps 100, open forceps 200 *(see* FIG. 9), or any other suitable surgical instrument and/or system may be utilized in accordance with the disclosure. Obviously, different electrical and mechanical connections and considerations apply to each particular type of instrument and system; however, the aspects and features of the disclosure remain generally consistent regardless of the configuration of the instrument or system used therewith.

Endoscopic forceps 100 defines a longitudinal axis "A-A" and includes a housing 120, a handle assembly 130, a rotating assembly 170, a trigger assembly 180, and an end effector assembly 10. Forceps 100 further includes a shaft 112 having a distal end 114 configured to mechanically engage end effector assembly 10 and a proximal end 116 that mechanically engages housing 120. Forceps 100 may further include a surgical cable extending therefrom and configured to connect forceps 100 to an generator 160 such that at least one of the electrically-conductive tissue treating surfaces 13, 14 of jaw members 11, 12 of end effector assembly 10 may be energized to treat tissue grasped therebetween, e.g., upon activation of activation switch 190.

With continued reference to FIG. 5, handle assembly 130 includes fixed handle 150 and a movable handle 140. Fixed handle 150 is integrally associated with housing 120 and handle 140 is movable relative to fixed handle 150. Rotating assembly 170 is rotatable in either direction about a longitudinal axis "A-A" to rotate end effector assembly 10 about longitudinal axis "A-A." Housing 120 houses the internal working components of forceps 100.

End effector assembly 10 is shown attached at distal end 114 of shaft 112 and includes a pair of opposing jaw members 11 and 12. Each of jaw members 11 and 12 includes an electrically-conductive tissue treating surface 13, 14, respectively, configured to grasp tissue therebetween and conduct energy therethrough to treat, *e.g.,* seal, tissue. End effector assembly 10 is designed as a unilateral assembly, *i.e.,* where jaw member 12 is fixed relative to shaft 112 and jaw member 11 is movable relative to shaft 112 and fixed jaw member 12. However, end effector assembly 10 may alternatively be configured as a bilateral assembly, *i.e.,* where both jaw member 11 and jaw member 12 are movable relative to one another and to shaft 112. In some embodiments, a knife assembly (not shown) is disposed within shaft 112, and a knife channel (not shown) is defined within one or both jaw members 11, 12 to permit reciprocation of a knife blade (not shown) therethrough, e.g., upon activation of trigger 182 of trigger assembly 180.

Continuing with reference to FIG. 5, movable handle 140 of handle assembly 130 is ultimately connected to a drive assembly (not shown) that, together, mechanically cooperate to impart movement of jaw members 11 and 12 between a spaced-apart position and an approximated position to grasp tissue between tissue treating surfaces 13 and 14 of jaw members 11, 12, respectively. As shown in FIG. 5, movable handle 140 is initially spaced-apart from fixed handle 150 and, correspondingly, jaw members 11, 12 are in the spaced-apart position. Movable handle 140 is depressible from this initial position to a depressed position corresponding to the approximated position of jaw members 11, 12.

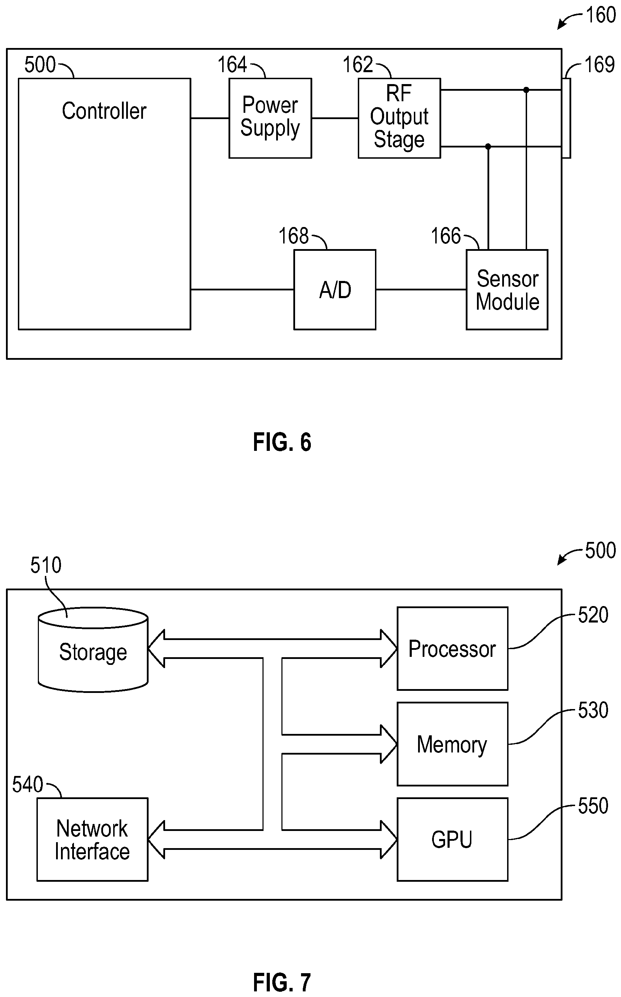

Referring now to FIG. 6, there is shown a block diagram of exemplary components of a generator 160 in accordance with aspects of the disclosure. In the illustrated embodiment, the generator 160 includes a controller 500, a power supply 164, a radio-frequency (RF) energy output stage 162, a sensor module 166, and one or more connector ports 169 that accommodate various types of energy-based surgical instruments. The generator 160 can include a user interface (not shown), which permits a user to select various parameters for the generator 160, such as mode of operation and power setting. In various embodiments, the power setting can be specified by a user to be between zero and a power limit, such as, for example, five watts, thirty watts, seventy watts, or ninety-five watts.

The generator 160 may be any suitable type of generator and may include a plurality of connectors to accommodate various types of energy-based surgical instruments (*e.g.,* monopolar energy-based surgical instrument and bipolar electrosurgical instrument). The generator 160 may also be configured to operate in a variety of modes, such as ablation, cutting, coagulation, and sealing. The generator 160 may include a switching mechanism (*e.g.,* relays) to switch the supply of RF energy among the connector ports 169 to which various energy-based surgical instruments may be connected. For example, when an energy-based surgical instrument, *e.g.,* forceps 100 (FIG. 5) or forceps 200 (FIG. 9), is connected to the generator 160, the switching mechanism switches the supply of RF energy to the monopolar port 169. In embodiments, the generator 160 may be configured to provide RF energy to a plurality of instruments simultaneously.

In various embodiments, the generator 160 may include a sensor module 166, which includes a plurality of sensors, *e.g.,* an RF current sensor and an RF voltage sensor. Various components of the generator 160, namely, the RF output stage 162 and the RF current and voltage sensors of sensor module 166 may be disposed on a printed circuit board (PCB). The RF current sensor of sensor module 166 may be coupled to the active terminal and provides measurements of the RF current supplied by the RF output stage 162. In embodiments, the RF current sensor of sensor module 166 may be coupled to the return terminal. The RF voltage sensor of sensor module 166 is coupled to the active and return terminals and provides measurements of the RF voltage supplied by the RF output stage 162. In embodiments, the RF current and voltage sensors of sensor module 166 may be coupled to active and return leads and, which interconnect the active and return terminals and to the RF output stage 162, respectively.

The sensed voltage and current from sensor module 166 are fed to analog-to-digital converters (ADCs) 168. The ADCs 168 sample the sensed voltage and current to obtain digital samples of the voltage and current of the RF output stage 162. The digital samples are processed by the controller 500 and used to generate a control signal to control the DC/AC inverter of the RF output stage 162 and the preamplifier. The ADCs 168 communicate the digital samples to the controller 500 for further processing.

In various embodiments, the controller 500 may collect data relating to the generator 160, including time, power, and/or impedance. For example, the energy-based surgical system may include a generator 160 and an energy-based surgical instrument such as detailed above with respect to FIG. 6. The generator 160 may be configured for electrical communication with the energy-based surgical instrument. While a surgeon is operating the energy-based surgical system during surgery, they may use the system to apply electrosurgical (RF) energy to tissue to treat tissue. More specifically, with additional reference to FIG. 6, tissue (not shown) is grasped between electrically conductive tissue treating surfaces 13, 14 of jaw members 11, 12 (or jaw members 21, 22 of FIG. 9) and electrosurgical (RF) energy is conducted between tissue treating surfaces 13, 14 and through tissue 302 to heat and thereby treat tissue. During such tissue treatment, the sensor circuitry, *e.g.,* sensor module 166, of the generator 160 may sense parameters of the tissue and/or energy such as, for example, impedance and power, and/or may supply data from which impedance and/or power can be derived such as for example, time, voltage, and/or current data. It is contemplated that pressure may also be sensed or determined. This may occur as a snapshot or over a time interval and may be determined at the beginning of tissue treatment, *e.g.,* at or within 250ms of initiation of tissue treatment. The sensed data may include, for example, time that the power is applied for, power applied to the tissue, and/or impedance of the tissue. The sensor module 166 may measure data from the energy-based surgical system, for example, the voltage and/or a current of the energy being delivered to the tissue. In various embodiments, the voltage and the current may be used to derive the power or the impedance. This sensed data obtained by the sensor circuitry is relayed to the controller 500 (via the ADC's 168, in embodiments) for further processing, as detailed below.

In various embodiments, the controller 500 uses the stored settings and the indication as training data for an artificial-intelligence learning system. In various embodiments, training and machine learning may be performed by a computing device outside of the generator 160, and the result of the machine learning may be communicated to the controller 500 of generator 160. In various embodiments, the controller 500 communicates the determined generator control parameter configuration that was output from the machine learning algorithm to a computing device, *e.g.,* of controller 500. In various embodiments, the artificial-intelligence learning system may use supervised learning, unsupervised learning, or reinforcement learning. In various embodiments, the neural network may include a temporal convolutional network, a fully connected network, or a feed forward network.

Referring to FIG. 7, a block diagram of exemplary components of a controller 500 is shown. The controller 500 includes a processor 520 connected to a computer-readable storage medium or a memory 530, which may be a volatile type memory, *e.g.,* RAM, or a non-volatile type memory, *e.g.,* flash media, disk media, *etc.* In various embodiments, the processor 520 may be another type of processor such as, without limitation, a digital signal processor, a microprocessor, an ASIC, a graphics processing unit (GPU), field-programmable gate array (FPGA), or a central processing unit (CPU). In various embodiments, network inference may also be accomplished in systems that may have weights implemented as memistors, chemically, or other inference calculations, as opposed to processors.

In various embodiments, the memory 530 can be random access memory, read only memory, magnetic disk memory, solid state memory, optical disc memory, and/or another type of memory. In various embodiments, the memory 530 can be separate from the controller 500 and can communicate with the processor 520 through communication buses of a circuit board and/or through communication cables such as serial ATA cables or other types of cables. The memory 530 includes computer-readable instructions that are executable by the processor 520 to operate the controller 500. In various embodiments, the controller 500 may include a network interface 540 to communicate with other computers or a server. In embodiments, a storage device 510 may be used for storing data. In various embodiments, the controller 500 may include one or more FPGAs 550. The FPGA 550 may be used for executing various machine learning algorithms such as those provided in accordance with the disclosure, as detailed below.

The memory 530 stores suitable instructions, to be executed by the processor 520, for receiving the sensed data, *e.g.,* sensed data from sensor module 166 via ADCs 168 (see FIG. 6), accessing storage device 510 of the controller 500, determining one or more tissue parameters, *e.g.,* tissue temperature, based upon the sensed data and information stored in storage device 510, and providing feedback based upon the determined tissue parameters. Storage device 510 of controller 500 stores one or more algorithms and/or models configured to estimate one or more tissue parameters, e.g., tissue temperature, based upon the sensed data received from sensory circuitry, e.g., from sensor module 166 via ADCs 168 (see FIG. 6). Although illustrated as part of generator 160, it is also contemplated that controller 500 be remote from generator 160, *e.g.,* on a remote server, and accessible by generator 160 via a wired or wireless connection. In embodiments where controller 500 is remote, it is contemplated that controller 500 may be accessible by and connected to multiple generators 160.

Referring now to FIG. 8, there is shown a flow diagram of a computer implemented method 800 for method for controlling an energy-based surgical procedure. Persons skilled in the art will appreciate that one or more operations of the method 800 may be performed in a different order, repeated, and/or omitted without departing from the scope of the invention as defined by the appended claims. In various embodiments, the illustrated method 800 can operate in the controller 500 (FIG. 7), in a remote device, or in another server or system. In various embodiments, some or all of the operations in the illustrated method 800 can operate using an energy-based surgical system, *e.g.,* instrument 100 or 200 and the generator 160 (see FIGS. 2 and 3). Other variations are contemplated to be within the scope of the disclosure. The operations of FIG. 8 will be described with respect to a controller, *e.g.,* controller 500 of generator 160 (FIGS. 6 and 7), but it will be understood that the illustrated operations are applicable to other systems and components thereof as well.

Initially, at step 802, the controller 500 may access an image of a tissue of a patient captured during an energy-based surgical procedure. In various embodiments, the image may be a video or a still image.

At step 804, the controller 500 accesses control parameter values of a generator 160, which provides energy. In various embodiments, the parameters may include time, slope, power, and/or impedance. In various embodiments, the controller 500 can also access patient parameters, which can be determined as described above in connection with FIGS. 6 and 7.

At step 806, controller 500 processes the image and the control parameters, and/or the patient parameters, by an artificial-intelligence learning system to provide an output relating to whether or not to maintain the current generator control parameter configuration. In various embodiments, the control parameters may include time, slope, power, and/or impedance.

At step 808, the controller 500 adjusts, automatically by the generator 160, the control parameters to provide adjusted energy based on the output of the artificial-intelligence learning system. In various embodiments, the controller 500 may deliver energy, by an energy-based surgical instrument configured to deliver energy to tissue using the adjusted energy. In various embodiments, the controller 500 may store settings of the energy-based surgical instrument based on the adjusted energy, in a memory of the generator 160. Thus, with the artificial intelligence learning system having been trained as detailed above, the system can determine whether to maintain a generator control parameter configuration for generating energy to treat the patient.

Referring now to FIG. 9, open forceps 200 is shown, including two elongated shafts 212a and 212b, each having a proximal end 216a and 216b, and a distal end 214a and 214b, respectively. Aspects of the disclosure with reference to FIG. 1-8, apply to the forceps 200 of FIG. 9. In particular, capturing an image of the surgical site. In various embodiments, one or more image sources may be used. As mentioned above, for example, the image capturing device may be located on a portion of the forceps 200. In various embodiments, the camera may be separate from the forceps 200, such as a camera located over the surgical site.

In various embodiments, the forceps 200 is a monopolar instrument. Forceps 200 is configured for use with an end effector assembly 20 that is similar to end effector assembly 10 of forceps 100 (see FIG. 5). More specifically, end effector assembly 20 is attached to distal ends 214a and 214b of shafts 212a and 212b, respectively, and includes a pair of opposing jaw members 21 and 22 that are movable relative to one another. Each shaft 212a and 212b includes a handle 217a and 217b disposed at the proximal end 216a and 216b thereof. Each handle 217a and 217b defines a finger hole 218a and 218b therethrough for receiving a finger of the user. As can be appreciated, finger holes 218a and 218b facilitate movement of shafts 212a and 212b relative to one another from an open position, wherein jaw members 21 and 22 are disposed in spaced-apart relation relative to one another, to a closed position, wherein jaw members 21 and 22 cooperate to grasp tissue therebetween.

A ratchet 230 may be included for selectively locking jaw members 21 and 22 of forceps 200 relative to one another at various different positions. It is envisioned that ratchet 230 may include graduations or other visual markings that enable the user to easily and quickly ascertain and control the amount of closure force desired between the jaw members 21 and 22.

With continued reference to FIG. 9, one of the shafts may be adapted to receive a surgical cable configured to connect forceps 200 to a power source (not shown). Alternatively, forceps 200 may be configured as a battery powered instrument having an internal or integrated power source (not shown). The power source (not shown), as will be described in greater detail below, provides power to end effector assembly 20 such that at least one of the electrically-conductive tissue treating surfaces 23, 24 of jaw members 21, 22, respectively, of end effector assembly 20 may be energized to treat tissue grasped therebetween.

Similar to forceps 100 (FIG. 5), forceps 200 may further include a knife assembly (not shown) disposed within either of shafts 212a, 212b and a knife channel (not shown) defined within one or both jaw members 21, 22 to permit reciprocation of a knife blade (not shown) therethrough.

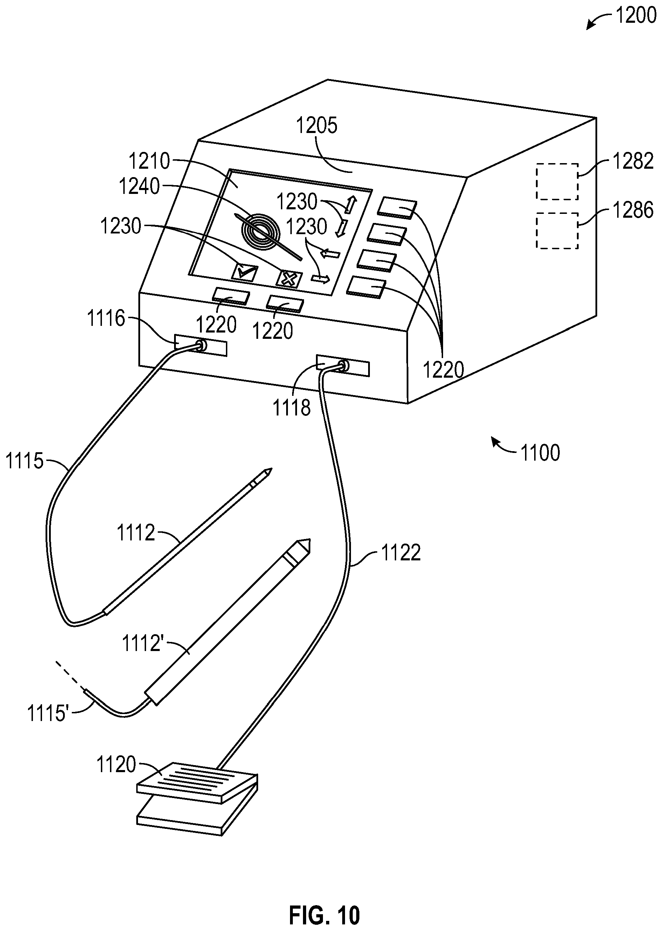

Referring now to FIG. 10, illustrated is a microwave ablation system 1100 in accordance with the present disclosure. The system 1100 includes a generator 1200, microwave antenna probe 1112 operably coupled by a cable 1115 via connector 1116 to the generator 1200, and an actuator 1120, which may be a footswitch, a hand-switch, a bite-activated switch, or any other suitable actuator. Actuator 1120 is operably coupled by a cable 1122 via connector 1118 to generator 1200. Cable 1122 may include one or more electrical conductors for conveying an actuation signal from actuator 1120 to generator 1200.

At least one additional or alternative microwave antenna probe 1112' may be included with microwave ablation system 1100 that may have characteristics distinct from that of microwave antenna probe 1112. For example, without limitation, microwave antenna probe 1112 may be a 12-gauge probe suitable for use with energy of about 915 MHz, while microwave antenna probe 1112' may be a 14-gauge probe suitable for use with energy of about 915 MHz. Other probe variations are contemplated within the scope of the present disclosure, for example, without limitation, a 12-gauge operable at 2450 MHz, and a 14 gauge operable at 2450 MHz. In use, the surgeon may interact with user interface 1205 of generator 1200 to preview operational characteristics of available probes 1112, 1112', and to choose a probe for use.

Generator 1200 includes a generator module 1286 that is configured as a source of microwave energy and is disposed in operable communication with processor 1282. In embodiments, generator module 1286 is configured to provide energy of about 915 MHz. Generator module 1286 may also be configured to provide energy of about 2450 MHz (2.45 GHz.). The present disclosure contemplates embodiments wherein generator module 1286 is configure to generate a frequency other than about 915 MHz or about 2450 MHz, and embodiments wherein generator module 1286 is configured to generate variable frequency energy. Probe 1112 is operably coupled to an energy output of generator module 1286.

Generator assembly 1200 also includes user interface 1205, that may include a display 1210 such as, without limitation, a flat panel graphic LCD display, adapted to visually display at least one user interface element 1230, 1240. In embodiments, display 1210 includes touchscreen capability (not explicitly shown), *e.g.,* the ability to receive input from an object in physical contact with the display, such as without limitation a stylus or a user's fingertip, as will be familiar to the skilled practitioner. A user interface element 1230, 1240 may have a corresponding active region, such that, by touching the screen within the active region associated with the user interface element, an input associated with the user interface element is received by the user interface 1205.

User interface 1205 may additionally or alternatively include one or more controls 1220, that may include without limitation a switch (*e.g.,* pushbutton switch, toggle switch, slide switch) and/or a continuous actuator (*e.g.,* rotary or linear potentiometer, rotary or linear encoder.) In embodiments, a control 1220 has a dedicated function, *e.g.,* display contrast, power on/off, and the like. Control 1220 may also have a function which may vary in accordance with an operational mode of the ablation system 1100. A user interface element 1230 may be positioned substantially adjacently to control 1220 to indicate the function thereof. Control 1220 may also include an indicator, such as an illuminated indicator (*e.g.,* a single- or variably-colored LED indicator).

It is contemplated that although FIGS. 9-12 above are exemplary, the instruments may be part of a robotic surgical system. Aspects, as described herein, apply to such a robotic surgery system as well.

From the foregoing and with reference to the various figure drawings, those skilled in the art will appreciate that certain modifications can also be made to the disclosure without departing from the scope of the invention as defined by the claims. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An energy-based surgical system comprising:
an image capturing device (102) configured to capture an image of tissue of a patient; and
a generator (160) configured to provide energy based on control parameters, the generator configured to execute instructions to perform a method including:
accessing the image of the tissue of the patient,
accessing control parameter values of the control parameters;
processing the image of the tissue and the control parameter values by an artificial-intelligence learning system to provide an output relating to configuration of the control parameters;
providing an indication to a clinician based on the output, the indication indicating whether to maintain the control parameter values; and
providing adjusted control parameter values for the generator based on the output of the artificial-intelligence learning system, if the indication indicates not to maintain the control parameter values
wherein the system further includes:
at least one of a user-interface or a voice recorder; and
an energy-based surgical instrument (100),
wherein the generator provides the energy to the energy-based surgical instrument for the clinician to apply to the tissue, and
wherein the at least one of the user-interface (1205) or the voice recorder receives information from the clinician on an outcome of applying the energy to the tissue.

2. The energy-based surgical system of claim 1, wherein the output of the artificial-intelligence learning system relates to a predicted outcome of applying the energy based on the control parameter values to the tissue.

3. The energy-based surgical system of claim 1, wherein the information from the clinician indicates one of: a successful outcome of applying the energy to the tissue or an unsuccessful outcome of applying the energy to the tissue.

4. The energy-based surgical system of claim 3, wherein the information from the clinician includes at least one of audio recorded by the voice recorder or a selection using the user-interface.

5. The energy-based surgical system of claim 1, wherein the image is an image of the tissue captured before the clinician applied the energy to the tissue, and wherein the method performed by executing the instructions in the generator further includes:
processing the information from the clinician to provide a tag for training the artificial-intelligence learning system;
storing training data including the control parameter values and the image of the tissue captured before the clinician applied the energy to the tissue; and
associating and storing the training data with the tag.

6. The energy-based surgical system of claim 1, further comprising a storage the includes patient information,
wherein the artificial-intelligence learning system is configured to provide the output relating to configuration of the control parameters based further on the patient information.

7. The energy-based surgical system of claim 6, wherein the patient information includes at least one of patient age, moisture of the tissue, hydration of the tissue, or location of the tissue.

8. The energy-based surgical system of claim 1, wherein the artificial-intelligence learning system includes a convolutional neural network that processes the image of the tissue.

9. The energy-based surgical system of claim 8, wherein the generator, in providing the adjusted control parameter values, executes the instructions to:
automatically adjust the control parameters; and
provide an indication to the clinician that the control parameters have been automatically adjusted.

10. A memory storing computer implemented method steps for an energy-based surgical procedure, comprising the steps of:
accessing an image (902) of tissue of a patient;
accessing control parameter values (904) of a generator configured to provide energy based on control parameters;
processing the image of the tissue and the control parameter values by an artificial-intelligence learning system (908) to provide an output relating to configuration of the control parameters;
providing an indication to a clinician based on the output, the indication indicating whether to maintain the control parameter values; and
providing adjusted control parameter values for the generator based on the output of the artificial-intelligence learning system, if the indication indicates not to maintain the control parameter values
wherein the energy-based surgical procedure further comprises:
providing the energy for the clinician to apply to the tissue;
applying the energy to the tissue using an energy-based surgical instrument; and
receiving information from the clinician on an outcome of applying the energy to the tissue.

11. The memory of claim 10, wherein the output of the artificial-intelligence learning system relates to a predicted outcome of applying the energy based on the control parameter values to the tissue.

12. The memory of claim 10, wherein the information from the clinician indicates one of: a successful outcome of applying the energy to the tissue or an unsuccessful outcome of applying the energy to the tissue.

13. The memory of claim 12, wherein the information from the clinician includes at least one of an audio recording or a user-interface selection.

14. The memory of claim 10, wherein the image is an image of the tissue captured before the clinician applied the energy to the tissue, and the method further comprising the steps of:
processing the information from the clinician to provide a tag for training the artificial-intelligence learning system;
storing training data including the control parameter values and the image of the tissue captured before the clinician applied the energy to the tissue; and
training the artificial-intelligence learning system based on the training data and the tag.

15. The memory of claim 10, further comprising the steps of accessing patient information,
wherein the artificial-intelligence learning system is configured to provide the output relating to configuration of the control parameters based further on the patient information.

## Patentansprüche

1. Energiebasiertes chirurgisches System, umfassend:
eine Bildaufnahmevorrichtung (102), die ausgelegt ist, um ein Bild von Gewebe eines Patienten aufzunehmen; und
einen Generator (160), der ausgelegt ist, um Energie basierend auf Steuerparametern bereitzustellen, wobei der Generator ausgelegt ist, um Anweisungen auszuführen, um ein Verfahren durchzuführen, das beinhaltet:
Zugreifen auf das Bild des Gewebes des Patienten,
Zugreifen auf Steuerparameterwerte der Steuerparameter;
Verarbeiten des Bildes des Gewebes und der Steuerparameterwerte durch ein Lernsystem mit künstlicher Intelligenz, um einen Ausgang bezüglich der Konfiguration der Steuerparameter bereitzustellen;
Bereitstellen einer Anzeige für einen Kliniker basierend auf dem Ausgang, wobei die Anzeige angibt, ob die Steuerparameterwerte beibehalten werden sollen; und
Bereitstellen angepasster Steuerparameterwerte für den Generator basierend auf dem Ausgang des Lernsystems mit künstlicher Intelligenz, wenn die Anzeige anzeigt, dass die Steuerparameterwerte nicht beibehalten werden sollen,
wobei das System ferner aufweist:
mindestens eine Benutzerschnittstelle oder ein Sprachaufzeichnungsgerät; und
ein energiebasiertes chirurgisches Instrument (100),
wobei der Generator die Energie an das energiebasierte chirurgische Instrument bereitstellt, damit der Kliniker sie auf das Gewebe anwenden kann, und
wobei die Benutzerschnittstelle (1205) und/oder das Sprachaufzeichnungsgerät vom Kliniker Informationen über das Ergebnis der Anwendung der Energie auf das Gewebe erhalten.

2. Energiebasiertes chirurgisches System nach Anspruch 1, wobei der Ausgang des Lernsystems mit künstlicher Intelligenz auf ein vorhergesagtes Ergebnis der Anwendung der Energie basierend auf den Steuerparameterwerten an das Gewebe bezogen ist.

3. Energiebasiertes chirurgisches System nach Anspruch 1, wobei die Informationen vom Kliniker eines der folgenden anzeigen: ein erfolgreiches Ergebnis der Anwendung der Energie auf das Gewebe oder ein nicht erfolgreiches Ergebnis der Anwendung der Energie auf das Gewebe.

4. Energiebasiertes chirurgisches System nach Anspruch 3, wobei die Informationen vom Kliniker mindestens eines von Audio, das vom Sprachaufzeichnungsgerät aufgezeichnet wurde, oder einer Auswahl über die Benutzerschnittstelle aufweisen.

5. Energiebasiertes chirurgisches System nach Anspruch 1, wobei das Bild ein Bild des Gewebes ist, das aufgenommen wurde, bevor der Kliniker die Energie auf das Gewebe angewendet hat, und wobei das Verfahren, das durch Ausführen der Anweisungen im Generator durchgeführt wird, ferner beinhaltet:
Verarbeiten der Informationen vom Kliniker, um ein Tag für das Training des Lernsystems mit künstlicher Intelligenz bereitzustellen;
Speichern von Trainingsdaten einschließlich der Steuerparameterwerte und des Bildes des Gewebes, das aufgenommen wurde, bevor der Kliniker die Energie auf das Gewebe angewendet hat; und
Verknüpfen und Speichern der Trainingsdaten mit dem Tag.

6. Energiebasiertes chirurgisches System nach Anspruch 1, ferner einen Speicher umfassend, der Patienteninformationen enthält,
wobei das Lernsystem mit künstlicher Intelligenz ausgelegt ist, um die Ausgabe bezüglich der Konfiguration der Steuerparameter ferner basierend auf den Patienteninformationen bereitzustellen.

7. Energiebasiertes chirurgisches System nach Anspruch 6, wobei die Patienteninformationen mindestens eines von Alter des Patienten, Feuchtigkeit des Gewebes, Hydratation des Gewebes oder Position des Gewebes umfassen.

8. Energiebasiertes chirurgisches System nach Anspruch 1, wobei das Lernsystem mit künstlicher Intelligenz ein neuronales Faltungsnetzwerk aufweist, das das Bild des Gewebes verarbeitet.

9. Energiebasiertes chirurgisches System nach Anspruch 8, wobei der Generator beim Bereitstellen der angepassten Steuerparameterwerte die Anweisungen ausführt zum:
automatischen Anpassen der Steuerparameter; und
Bereitstellen einer Anzeige für den Kliniker, dass die Steuerparameter automatisch angepasst wurden.

10. Speicher, der computerimplementierte Verfahrensschritte für ein energiebasiertes chirurgisches Verfahren speichert, umfassend die Schritte:
Zugreifen auf ein Bild (902) von Gewebe eines Patienten;
Zugreifen auf Steuerparameterwerte (904) eines Generators, der ausgelegt ist, um Energie basierend auf Steuerparametern bereitzustellen;
Verarbeiten des Bildes des Gewebes und der Steuerparameterwerte durch ein Lernsystem mit künstlicher Intelligenz (908), um einen Ausgang bezüglich der Konfiguration der Steuerparameter bereitzustellen;
Bereitstellen einer Anzeige für einen Kliniker basierend auf dem Ausgang, wobei die Anzeige angibt, ob die Steuerparameterwerte beibehalten werden sollen; und
Bereitstellen angepasster Steuerparameterwerte für den Generator basierend auf dem Ausgang des Lernsystems mit künstlicher Intelligenz, wenn die Anzeige anzeigt, dass die Steuerparameterwerte nicht beibehalten werden sollen,
wobei das energiebasierte chirurgische Verfahren ferner umfasst:
Bereitstellen der auf das Gewebe anzuwendenden Energie an den Kliniker;
Anwenden der Energie auf das Gewebe mittels eines energiebasierten chirurgischen Instruments; und
Empfangen von Informationen vom Kliniker über ein Ergebnis des Anwendung der Energie auf das Gewebe.

11. Speicher nach Anspruch 10, wobei der Ausgang des Lernsystems mit künstlicher Intelligenz auf ein vorhergesagtes Ergebnis der Anwendung der Energie basierend auf den Steuerparameterwerten an das Gewebe bezogen ist.

12. Speicher nach Anspruch 10, wobei die Informationen vom Kliniker eines der folgenden anzeigen: ein erfolgreiches Ergebnis der Anwendung der Energie auf das Gewebe oder ein nicht erfolgreiches Ergebnis der Anwendung der Energie auf das Gewebe.

13. Speicher nach Anspruch 12, wobei die Informationen vom Kliniker mindestens eines von einer Audio-Aufzeichnung oder einer Auswahl über eine Benutzerschnittstelle aufweisen.

14. Speicher nach Anspruch 10, wobei das Bild ein Bild des Gewebes ist, das aufgenommen wurde, bevor der Kliniker die Energie auf das Gewebe angewendet hat, und wobei das Verfahren ferner die Schritte umfasst:
Verarbeiten der Informationen vom Kliniker, um ein Tag für das Training des Lernsystems mit künstlicher Intelligenz bereitzustellen;
Speichern von Trainingsdaten einschließlich der Steuerparameterwerte und des Bildes des Gewebes, das aufgenommen wurde, bevor der Kliniker die Energie auf das Gewebe angewendet hat; und
Trainieren des Lernsystems mit künstlicher Intelligenz basierend auf den Trainingsdaten und dem Tag.

15. Speicher nach Anspruch 10, ferner die Schritte des Zugreifens auf Patienteninformationen umfassend,
wobei das Lernsystem mit künstlicher Intelligenz ausgelegt ist, um die Ausgabe bezüglich der Konfiguration der Steuerparameter ferner basierend auf den Patienteninformationen bereitzustellen.

## Revendications

1. Système chirurgical à base d'énergie comprenant :
un dispositif de capture d'image (102) configuré pour capturer une image de tissu d'un patient ; et
un générateur (160) configuré pour fournir de l'énergie sur la base de paramètres de commande, le générateur étant configuré pour exécuter des instructions pour réaliser un procédé comprenant :
l'accès à l'image du tissu du patient,
l'accès à des valeurs de paramètres de commande des paramètres de commande ;
le traitement de l'image du tissu et des valeurs des paramètres de commande par un système d'apprentissage à intelligence artificielle pour fournir une sortie relative à la configuration des paramètres de commande ;
la fourniture d'une indication à un clinicien sur la base de la sortie, l'indication indiquant s'il faut maintenir les valeurs des paramètres de commande ; et
la fourniture des valeurs de paramètres de commande ajustées pour le générateur sur la base de la sortie du système d'apprentissage à intelligence artificielle, si l'indication indique de ne pas maintenir les valeurs de paramètres de commande
le système comprenant en outre :
au moins l'un parmi une interface utilisateur ou un enregistreur vocal ; et
un instrument chirurgical à base d'énergie (100),
le générateur fournissant l'énergie à l'instrument chirurgical à base d'énergie pour que le clinicien l'applique au tissu, et
l'au moins un parmi l'interface utilisateur (1205) ou l'enregistreur vocal recevant des informations provenant du clinicien sur le résultat de l'application de l'énergie au tissu.

2. Système chirurgical à base d'énergie selon la revendication 1, la sortie du système d'apprentissage à intelligence artificielle
concernant un résultat prédit de l'application de l'énergie sur la base des valeurs des paramètres de commande au tissu.

3. Système chirurgical à base d'énergie selon la revendication 1, les informations provenant du clinicien indiquant un résultat parmi : un résultat réussi d'application de l'énergie au tissu ou un résultat non réussi d'application de l'énergie au tissu.

4. Système chirurgical à base d'énergie selon la revendication 3, les informations provenant du clinicien comprenant au moins l'un d'un signal audio enregistré par l'enregistreur vocal ou d'une sélection utilisant l'interface utilisateur.

5. Système chirurgical à base d'énergie selon la revendication 1, l'image étant une image du tissu capturée avant que le clinicien n'applique l'énergie au tissu, et le procédé effectué en exécutant les instructions dans le générateur comprenant en outre :
le traitement des informations provenant du clinicien pour fournir une étiquette pour la formation du système d'apprentissage à intelligence artificielle ;
le stockage des données d'apprentissage comprenant les valeurs des paramètres de commande et l'image du tissu capturée avant que le clinicien n'applique l'énergie au tissu ; et
l'association et le stockage des données d'apprentissage sur l'étiquette.

6. Système chirurgical à base d'énergie selon la revendication 1, comprenant en outre une mémoire qui inclut des informations sur le patient,
le système d'apprentissage à intelligence artificielle étant configuré pour fournir la sortie relative à la configuration des paramètres de commande sur la base en outre des informations sur le patient.

7. Système chirurgical à base d'énergie selon la revendication 6, les informations relatives au patient comprenant au moins un élément parmi l'âge du patient, l'humidité du tissu, l'hydratation du tissu, ou l'emplacement du tissu.

8. Système chirurgical à base d'énergie selon la revendication 1, le système d'apprentissage à intelligence artificielle comprenant un réseau neuronal convolutif qui traite l'image du tissu.

9. Système chirurgical à base d'énergie selon la revendication 8, le générateur, en fournissant les valeurs de paramètres de commande ajustées, exécute les instructions pour :
régler automatiquement les paramètres de commande ; et
indiquer au clinicien que les paramètres de commande ont été automatiquement réglés.

10. Mémoire stockant des étapes de procédé mises en œuvre par ordinateur pour une intervention chirurgicale à base d'énergie, comprenant les étapes de :
l'accès à une image (902) de tissu d'un patient :
l'accès à des valeurs de paramètres de commande (904) d'un générateur configuré pour fournir de l'énergie sur la base de paramètres de commande ;
le traitement de l'image du tissu et des valeurs des paramètres de commande par un système d'apprentissage à intelligence artificielle (908) pour fournir une sortie relative à la configuration des paramètres de commande ;
la fourniture d'une indication à un clinicien sur la base de la sortie, l'indication indiquant s'il faut maintenir les valeurs des paramètres de commande ; et
la fourniture des valeurs de paramètres de commande ajustées pour le générateur sur la base de la sortie du système d'apprentissage à intelligence artificielle, si l'indication indique de ne pas maintenir les valeurs de paramètres de commande
l'intervention chirurgicale à base d'énergie comprenant en outre :
la fourniture de l'énergie que le clinicien doit appliquer au tissu ;
l'application de l'énergie au tissu à l'aide d'un instrument chirurgical à base d'énergie ; et
la réception d'informations provenant du clinicien sur le résultat de l'application de l'énergie aux tissus.

11. Mémoire selon la revendication 10, la sortie du système d'apprentissage à intelligence artificielle concernant un résultat prédit d'application de l'énergie sur la base des valeurs de paramètres de commande au tissu.

12. Mémoire selon la revendication 10, les informations provenant du clinicien indiquant un résultat parmi : un résultat réussi d'application de l'énergie au tissu ou un résultat non réussi d'application de l'énergie au tissu.

13. Mémoire selon la revendication 12, les informations provenant du clinicien comprenant au moins l'un d'un enregistrement audio ou d'une sélection d'interface utilisateur.

14. Mémoire selon la revendication 10, l'image étant une image du tissu capturé avant que le clinicien applique l'énergie au tissu, et le procédé comprenant en outre les étapes suivantes :
le traitement des informations provenant du clinicien pour fournir une étiquette pour la formation du système d'apprentissage à intelligence artificielle ;
le stockage des données d'apprentissage comprenant les valeurs des paramètres de commande et l'image du tissu capturée avant que le clinicien n'applique l'énergie au tissu ; et
la formation du système d'apprentissage à intelligence artificielle à partir des données d'apprentissage et de l'étiquette.

15. Mémoire selon la revendication 10, comprenant en outre les étapes consistant à accéder à des informations de patient,
le système d'apprentissage à intelligence artificielle étant configuré pour fournir la sortie relative à la configuration des paramètres de commande sur la base en outre des informations sur le patient.
